(19) Europäisches Patentamt
European Patent Office
Office européen des brevets

(11) Publication number: 0 338 992 A2

# (12) EUROPEAN PATENT APPLICATION

(21) Application number: **89810287.6**

(22) Date of filing: **17.04.89**

(51) Int. Cl.⁴: **C 07 C 79/36**
C 07 C 49/813,
C 07 C 49/835, C 07 C 49/84,
A 01 N 43/54, A 01 N 43/90,
A 01 N 35/06, A 01 N 43/14,
C 07 C 143/68,
C 07 C 149/32, C 07 C 147/06

(30) Priority: **18.04.88 US 182534**

(43) Date of publication of application:
**25.10.89 Bulletin 89/43**

(84) Designated Contracting States:
**AT BE CH DE ES FR GB GR IT LI LU NL SE**

(71) Applicant: **SANDOZ AG**
**Lichtstrasse 35**
**CH-4002 Basel (CH)**

(84) Designated Contracting States:
**BE CH ES FR GB GR IT LI LU NL SE**

(71) Applicant: **SANDOZ-PATENT-GMBH**
**Humboldtstrasse 3**
**D-7850 Lörrach (DE)**

(84) Designated Contracting States: **DE**

(71) Applicant: **SANDOZ-ERFINDUNGEN**
**Verwaltungsgesellschaft m.b.H.**
**Brunner Strasse 59**
**A-1235 Wien (AT)**

(84) Designated Contracting States: **AT**

(72) Inventor: **Lee, Shy-Fuh**
**228 Carbonera Avenue**
**Sunnyvale California 94086 (US)**

(54) **Improvements in or relating to organic compounds.**

(57) Compounds of the general formula

$$A-\overset{\overset{O}{\|}}{C}-B$$

wherein A is a bicyclic 1-one-2-ene-3-ol-2-yl residue;
B is an aryl or heteroaryl group optionally bearing substituents; and salts, enolethers and enolesters thereof, and methods of producing such compounds and using such compounds as herbicides are disclosed.

EP 0 338 992 A2

## Description

# IMPROVEMENTS IN OR RELATING TO ORGANIC COMPOUNDS

This invention relates to novel substituted aryl or heteroaryl bicyclodiones and related compounds, intermediates thereof, synthesis thereof, and the use of said compounds for the control of weeds.

Cyclic 1-one-2-ene-3-ol compounds substituted in the 2-position by an aroyl or heteroaroyl group and enol ethers and enol esters thereof are known herbicidal compounds. It has been found that 2-aroyl and 2-heteroaroyl-(bicyclic 1-one-2-ene-3-ol) compounds, as well as enol ethers and enolesters thereof have interesting biological properties. They are indicated for agricultural use, in particular for the control of undesired vegetation.

More particularly, this invention concerns compounds of the general formula

$$A-\overset{\overset{O}{\|}}{C}-B$$

wherein A is a bicyclic 1-one-2-ene-3-ol-2yl residue;
B is an aryl or heteroaryl group optionally bearing substituents; and salts, enolethers and enolesters thereof.

A particular group of compounds is that represented by formula I

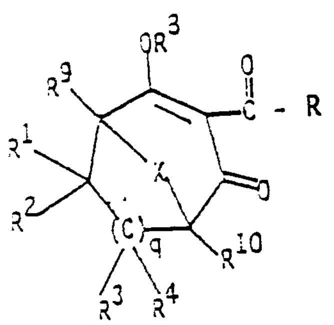

(I)

wherein, R is selected from the groups

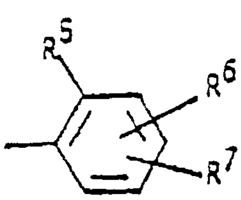

or

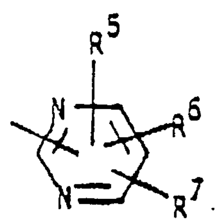

;

X is oxygen, sulfur or C1-4alkylene;
each of $R_1$, $R_2$, $R_3$, $R_4$, $R_9$ and $R_{10}$ is, independently, hydrogen, $C_{1-8}$alkyl or $COOR_{16}$;
$R_5$ is $C_{1-8}$alkyl, optionally substituted with one to six halogen atoms; $C_{1-8}$alkoxy, optionally substituted with one to six halogen atoms; $C_{1-8}$alkylcarbonyl; $C_{1-8}$alkoxycarbonyl; $NR_{13}R_{14}$; $O_nS(O)_{n'}R_{12}$; halogen; $NR_{15}SO_2R_{12}$; cyano; or nitro;
each of $R_6$ and $R_7$ is independently hydrogen or selected from the values of $R_5$; or $R_6$ and $R_7$ together from the group -Y-W-Z-;
$R_8$ is hydrogen, $C_{1-8}$alkyl, $C_{1-8}$alkylcarbonyl, $C_{1-8}$alkoxycarbonyl $C(O)NR_{13}R_{14}$, $C_{1-8}$alkylsulfonyl, $P(O)-(OR_{11})_2$, $R_{13}P(O)OR_{11}$, benzoyl or a salt forming moiety;
$R_{11}$ is $C_{1-8}$alkyl;
$R_{12}$ is $C_{1-8}$alkyl, optionally substituted with one to six halogen atoms;
each of $R_{13}$, $R_{14}$, $R_{15}$ and $R_{16}$ is, independently, hydrogen or $C_{1-8}$alkyl;
each of $R_{17}$ and $R_{18}$ is independently hydrogen, halogen or $C_{1-8}$alkyl optionally substituted with one to six halogen atoms; or $R_{17}$ and $R_{18}$ together form an oxo group;
each of $R_{19}$ and $R_{20}$ is, independently, hydrogen, halogen or C1-8alkyl optionally substituted with one to six halogen atoms;
W is $-(CR_{17}R_{18})_t-(CR_{19}R_{20})_{t'}-$ or $SO_2$;
each of Y and Z is independently oxygen, sulfur, $SO_2$, C=O or $CR_{15}R_{16}$; with the proviso that Y and Z are attached to adjacent carbons;
n is zero or one;
n′ is zero, one or two;
q is zero, one or two;
t is one or two; and
t′ is zero or one.

Enol compounds of the formula (I), wherein $R_8$ is H, can exist in a number of tautomeric forms, the following being representative;

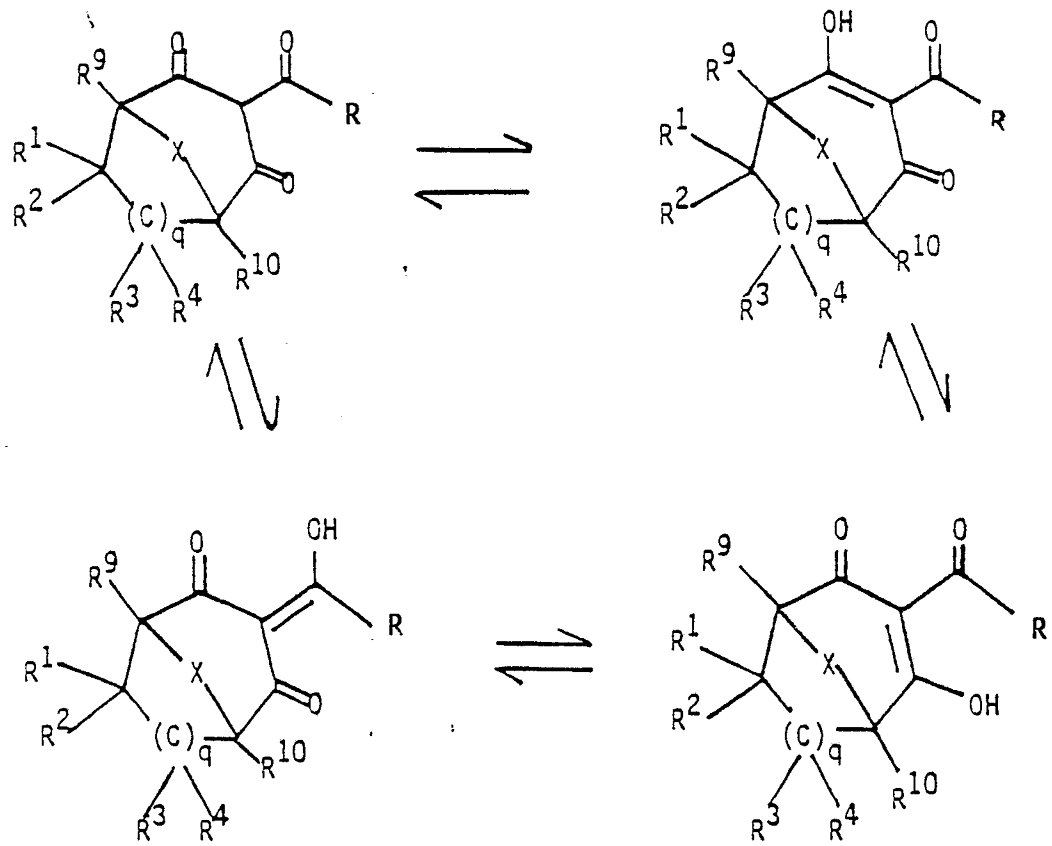

It is intended that all such tautomeric structures are included within the scope of this invention.

In the description and claims hereinafter, each of W, X, Y, Z, n, n′, q, t, t′ and $R_{1-20}$ is as defined above, unless otherwise specified.

In the practice of the present invention, X is preferably oxygen or $C_{1-2}$alkyl, more preferably $C_{1-2}$alkyl.

R is preferably substituted phenyl.

Where any of the substituents $R_5$-$R_7$, $R_{12}$ and $R_{17}$-$R_{20}$ is or comprises halogen, such halogen is conveniently selected from bromo, chloro and fluoro.

Where any of $R_1$-$R_{20}$ is or comprises $C_{1-8}$alkyl, it is preferably of one to four carbons.

Where any of $R_5$-$R_8$ is or comprises $C_{1-8}$alkoxy, it is preferably of one to four carbons.

Each of $R_1$-$R_4$, $R_9$ and $R_{10}$ is preferably hydrogen or $C_{1-4}$alkyl; such alkyl is more preferably of one to three carbons; each of $R_1$-$R_4$, $R_9$ and $R_{10}$ is more preferably hydrogen.

$R_5$ conveniently signifies $C_{1-4}$alkyl optionally substituted with halogen, $-(O)_n-S(O)_{n'}C_{1-4}$alkyl, halogen or nitro. It is preferably methyl, $CF_3$, $C_{1-3}$alkylsulfonyl, $C_{1-3}$alkylsufonyloxy, chloro, bromo or nitro, more preferably nitro.

$R_6$ is conveniently hydrogen, $C_{1-4}$alkyl, $C_{1-4}$alkoxy, bromo, chloro or, together with $R_7$, the group -Y-W-Z-; it is preferably hydrogen, methoxy or chloro, or, together with $R_7$, methylenedioxy; more preferably hydrogen.

$R_7$ is preferably bromo, chloro, fluoro, $OSO_2C_{1-4}$alkyl, $SO_2C_{1-4}$alkyl, $OSO_2C_{1-4}$haloalkyl, $SC_{1-4}$alkyl, $NR_{15}SO_2C_{1-4}$alkyl, or, together with $R_6$, the group -Y-W-Z-. It is more preferably chloro, fluoro, bromo, $C_{1-3}$alkylthio, $C_{1-3}$alkylsufonyl or $C_{1-3}$alkylsulfonyloxy.

$R_8$ is conveniently hydrogen, $C_{1-4}$alkyl, $C_{4-8}$alkylcarbonyl, benzoyl or $C_{1-4}$alkylsulfonyl, It is preferably hydrogen, methyl, ethyl, t-butylcarbonyl, isobutylcarbonyl, benzoyl or methylsulfonyl, more preferably hydrogen.

Where $R_8$ is a salt forming moiety, it is preferably inorganic e.g. a metal equivalent of Na, Ca, Fe or Cu; or organic, e.g., the ammonium salt moiety of an amine (such as 1-(methylaminomethyl)-naphthalene), a sulfonium, sulfoxomium of phosphonium moiety. Depending on the nature of $R_8$, the salt may exist in chelated form.

q is preferably 1 or 2.

Preferably $R_6$ is in the 3-position and $R_7$ is in the 4-position.

The compounds of the present invention of formula I are new substances which can be prepared by

methods analogous to methods known in the art for the preparation of 2-aroyl-(bicyclic-1,3-diones) and enol ethers or enol esters thereof.

More particularly, they can be obtained by, for example: reacting an enol ester of formula (II)

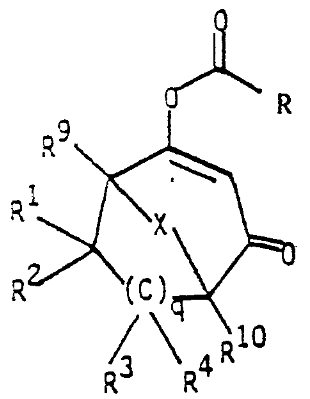

(II)

wherein R, X, q, $R_1$-$R_4$ and $R_9$-$R_{10}$ are as defined above, with a cyanide source and a moderate base to give a compound of formula I where $R_8$ is hydrogen, followed, where desired, by etherification or esterification to the corresponding enol ethers or enol esters.

The above reaction is carried out in the presence of a catalytic amount of a source of cyanide anion and/or hydrogen cyanide, together with a molar excess, with respect to the enol ester, or a moderate base. The reaction is conveniently carried out in a solvent which is inert under the reaction conditions, e.g. 1,2-dichloroethane, toluene, acetonitrile, methylene chloride, ethyl acetate, dimethylformamide (DMF) and methyl isobutyl ketone (MIBK). In general, depending on the nature of the reactants and the cyanide source, the rearrangement may be conducted at temperatures up to about 80° C. In some cases, for instance when there is a possible problem of excessive by-product formation, the temperatures should be kept at about 40° C maximum.

Preferred cyanide sources are alkali metal cyanides such as sodium and potassium cyanide; cyanohydrins of methyl alkyl ketones having from 1-4 carbon atoms in the alkyl groups, such as acetone or methyl isobutyl ketone cyanohydrins; cyanohydrins of benzaldehyde or of $C_2$-$C_5$ aliphatic aldehydes such as acetaldehyde, propionaldehyde, etc., cyanohydrins; zinc cyanide; tri(lower alkyl) silyl cyanides, notably trimethyl silyl cyanide; and hydrogen cyanide itself. Among cyanohydrins the preferred cyanide source is acetone cyanohydrin. The cyanide source is used in an amount up to about 50 mole percent based on the enol ester. Generally about 1-10 mole % of the cyanide source is preferred.

By the term "moderate base" is meant a substance which acts as a base yet whose strength or activity as a base lies between that of strong bases such as hydroxides (which could cause hydrolysis of the enol ester) and that of weak bases such as bicarbonates (which would not function effectively). Moderate bases suitable for use in this reaction include both organic bases such as tertiary amines and inorganic bases such as alkali metal carbonates and phosphates. Suitable tertiary amines include trialkylamines such as triethylamine, trialkanolamines such as triethanolamine, and pyridine. Suitable inorganic bases include potassium carbonate and trisodium phosphate. The base is used in an amount of from about 1 to about 4 moles per mole of enol ester, preferably about 1.3-2 moles per mole.

When the cyanide source is an alkali metal cyanide, particularly potassium cyanide, a phase transfer catalyst may be included in the reaction. Particularly suitable phase transfer catalysts are the Crown ethers.

Depending on the reaction conditions, the thus obtained keto-enol compounds may be in free acid form ($R_8$ is H) or in salt form; where they are in salt form (i.e. $R_8$ is a salt forming moiety), $R_8$ may be inorganic (e.g. a metal equivalent of Na, Ca, Fe or Cu) or organic, e.g. the ammonium salt moiety of an amine, sulfonium, sulfoxomium or phosphonium moiety. Depending on the nature of $R_8$, the salt may exist in chelated form. The salt form may be converted to the corresponding acid form ($R_8$ is H) in a manner known per se, and vice versa.

Compounds of formula I where $R_8$ is other than hydrogen or a salt forming moiety can be prepared in a manner known per se for the preparation of enol ethers or enol esters from the corresponding enol compounds, e.g. by reacting a compound of formula I where $R_8$ = H with either

a) the group $R_8$-OH and a catalyst, or

b) the group $R_8$-Q and a moderate base, wherein Q is a halogen atom, to give a compound of formula I where $R_8$ is as defined above other than hydrogen or a salt forming moiety.

The above reaction a) is carried out in the presence of a catalyst such as concentrated sulfuric acid. The reaction is conveniently carried out in a solvent which is also the reactant such as methanol, and at an elevated temperature.

The above reaction b) is carried out in the presence of a moderate base such as triethylamine or pyridine and conveniently at RT or below.

The compounds of formula I may be recovered from the reaction mixture in which they are formed by working up by established procedures.

The starting materials and reagents employed in the process described herein are either known or, insofar

as they are not known, may be produced in a manner analogous to the processes described herein or to known processes.

The novel compounds of formula I are useful for the control of weeds, using pre- and/or post-emergent treatments. Some of them are also useful as plant growth regulators and as acaricides. Application of a compound of the present invention as a herbicide is made according to conventional procedure to the weeds or their locus using an herbicidally effective amount of the compounds, usually from about one-half or less to ten pounds per acre.

The term "herbicide", as used herein, refers to an active ingredient which modifies the growth of plants because of phytotoxic or plant growth regulating properties so as to retard the growth of the plant or damage the plant sufficiently to kill it.

The compounds of the present invention, when applied as either post or pre-emergents, demonstrate high levels of herbicidal activity on broadleaf, grass and sedge weeds.

In the use of the compounds of formula I as a herbicide or acaricide, a compound of formula I, or mixtures thereof, can conveniently be employed as agricultural compositions in association with acceptable diluent(s) for application to the weed, acari, or their loci. Such compositions also form part of the present invention.

Methods of preparing suitable compositions which can be used with a compound of the present invention are described in the literature along with suitable liquid and solid carriers. The optimum usage of a compound of the present invention is readily determinable by one of ordinary skill in the art using routine testing such as greenhouse testing and small plot testing.

Suitable compositions contain from 0.01 to 99 % by weight of active ingredient, from 0 to 20 % of surfactant and from 1 to 99.99 % of solid or liquid diluent(s). Higher ratios of surfactant to active ingredient are sometimes desirable and are achieved by incorporation into the formulation or by tank mixing. Application forms of a composition generally contain between 0.01 and 25 % by weight of active ingredient. Lower or higher levels of active ingredient can, of course, be present depending on the intended use, the physical properties of the compound and the mode of application. Concentrate forms of a composition intended to be diluted before use generally contain between 2 and 90 %, preferably between 5 and 81 % by weight of active ingredient.

Useful compositions containing the compound of formula I include dusts, granules, suspension concentrates, wettable powders, flowables and the like. They are obtained by conventional manner, e.g. by mixing a compound of formula I with the diluent(s) and optionally with other ingredients.

## EXAMPLE A

### Preparation of a Dust

10 Parts of a compound according to this invention and 90 parts of powdered talc are mixed in a mechanical grinder-blender and are ground until a homogeneous, free-flowing dust of the desired particle size is obtained. This dust is suitable for direct application to the site of the weed,acari infestation.

## EXAMPLE B

### Preparation of Wettable Powder

25 Parts of a compound according to this invention are mixed and milled with 25 parts of synthetic fine silica, 2 parts of sodium lauryl sulphate, 3 parts of sodium ligninsulphonate and 45 parts of finely divided kaolin until the mean particle size is about 5 micron. The resulting wettable powder is diluted with water before use to a spray liquor with the desired concentration.

## EXAMPLE C

### Preparation of Emulsifiable Concentrate (EC)

13.37 Parts of a compound according to this invention are mixed in a beaker with 7.04 parts of Toximul 360A (a mixture of anionic and non-ionic surfactants containing largely anionic surfactants), 23.79 parts of dimethylformamide and 55.8 parts of Tenneco 500-100 (predominantly a mixture of alkylated aromatics such as xylene and ethylbenzene) until solution is effected. The resulting EC is diluted with water for use.

Alternatively, the compounds of formula I may be used in micro-encapsulated form.

The compounds of formula I can be combined with a cyclodextrin to make a cyclodextrin inclusion complex for application to the pest or its locus.

Agriculturally acceptable additives may be employed in the compositions to improve the performance of the active ingredient and to reduce the foaming, caking and corrosion, for example.

"Surfactant" as used herein means an agriculturally acceptable material which imparts emulsifiability, spreading, wetting, dispersibility or other surface-modifying properties. Examples of surfactants are sodium lignin sulfonate and lauryl sulfate.

"Diluent" as used herein means a liquid or solid agriculturally acceptable material sued to dilute a concentrated material to a usable or desirable strength. For dusts or granules it can be e.g. talc, kaolin or diatomaceous earth, for liquid concentrate forms for example a hydrocarbon such as xylene or an alcohol such as isopropanol, and for liquid application forms e.g. water or diesel oil.

The compositions of this invention can also comprise other compounds having biological activity, e.g. compounds having similar or complementary herbicidal activity for broadspectrum weed control or compounds having acaricidal activity or compounds having antidotal, fungicidal, insecticidal or insect attractant activity.

The following examples are provided to illustrate the practice of the present invention. Temperature is given in degrees Centigrade. RT means room temperature. Parts and percentages are by weight.

PREPARATION OF FINAL COMPOUNDS

EXAMPLE 1

To a solution of 4-(4-chloro-2-nitrobenzoyloxy)-bicyclo-[3,2,1]-3-octen-2-one (0.75 g) in 12 ml of acetonitrile are added triethylamine (0.62 ml) and acetone cyanohydrin (0.15 ml) in one portion. The mixture is stirred at RT overnight, after which the acetonitrile is removed and the residue is taken up in methylene chloride, then washed with dil. HCl and with brine, dried and evaporated to dryness. The crude product is purified by PTLC to give 3-(4-chloro-2-nitrobenzoyl)-bicyclo-[3,2,1]octane-2,4-dione (compound 1, Table A).

EXAMPLE 2

To a solution of 4-(4-chloro-2-nitrobenzoyloxy)-5,8,8-trimethylbicyclo[3,2,1]-3-octen-2-one (4.20 g) in 25 ml of acetonitrile are added triethylamine (3.2 ml, 2 eq.) and acetone cyanohydrin (0.4 ml), and the mixture is stirred at RT for 16 hr. The acetonitrile is removed, and the residue is concentrated and purified by PTLC to give 3-(4-chloro-2-nitro-benzoyl)-1,8,8-trimethylbicyclo[3,2,1]octane-2,4-dione (compound 12, Table A).

EXAMPLE 3

To a solution of 4-(3-methoxy-4-methylsulfonyloxy-2-nitrobenzoyloxy)bicyclo[3,2,1]-3-octen-2-one (17.6 mmol) in 50 ml of acetonitrile are added triethylamine (4.91 ml, 2 eq.) and acetone cyanohydrin (1.0 ml) in one portion. The mixture is stirred at RT overnight, after which it is diluted with water and extracted with ether. The ether is removed and the residue is purified by PTLC to give 3-(3-methoxy-4-methylsulfonyloxy-2-nitrobenzoyl)-bicyclo[3,2,1]octane-2,4-dione (compound 9, Table A).

EXAMPLE 4

To a solution of 4-(4-methylsulfonyloxy-2-chlorobenzoyl-oxy) bicyclo[3,2,1]-3-octen-2-one (14.3 mmol) in 25 ml of acetonitrile are added triethylamine (3.97 ml, 2 eq.) and acetone cyanohydrin (0.4 ml), and the mixture is stirred at RT overnight. The acetonitrile is removed, and the residue is taken up in water and extracted with methylene chloride. The combined organic extracts are washed with dilute HCl and with brine, dried and evaporated to dryness. The crude product is purified from ether to give 3-(4-methylsulfonyloxy-2-chloro-benzoyl) bicyclo-[3,2,1]octane-2,4-dione (compound 4, Table A).

EXAMPLE 5

To a solution of 4-(3,4-methylenedioxy-2-nitrobenzoyloxy)-bicyclo[3,2,1]-3-octen-2-one (13.2 mmol) in 50 ml of acetonitrile are added triethylamine (3.69 ml) and acetone cyanohydrin (0.5 ml) in one portion. The mixture is stirred at RT for 2 days, after which it is diluted with water and extracted with ether. The ether is removed and the residue is purified by PTLC to give 3-(3,4-methylenedioxy-2-nitrobenzoyl)-bicyclo[3,2,1]-octane-2,4-dione (compound 11, Table A).

EXAMPLE 6

To a solution of 4-(4,5-methylenedioxy-2-nitrobenzoyl-oxy)-bicyclo[3,2,1]-3-octen-2-one (14.4 mmol) in acetonitrile are added triethylamine (4.0 ml, 28.8 mmol) and acetone cyanohydrin (0.4 ml), and the mixture is stirred at RT overnight. The acetonitrile is removed, and the residue is taken up in water and extracted with methylene chloride. The combined organic extracts are washed with dilute HCl and with brine, dried and evaporated to dryness. The crude product is purified by chromatography to give 3-(4,5-methylenedioxy-2-nitrobenzoyl)-bicyclo[3,2,1]octane-2,4-dione.

EXAMPLE 7

Following the procedure of any of Examples 1 through 6, each of the compounds under Table A is prepared by rearrangement of the corresponding enol ester.

EXAMPLE 8

A solution of 3-(4-chloro-2-nitrobenzoyl)-bicyclo[3,2,1]-octane-2,4-dione (2.54 mmol) and 2 drops of conc. sulfuric acid in 20 ml of methanol is heated under reflux for 48 hours. The reaction mixture is concentrated and the residue is taken up in ether. The ethereal solution is washed with aqueous sodium bicarbonate and with brine, dried and evaporated to dryness to give 2-methoxy-3-(4-chloro-2-nitrobenzoyl)-bicyclo[3,2,1]-octane-4-one.

EXAMPLE 9

To a mixture of 3-(4-chloro-2-nitrobenzoyl)-bicyclo[3,2,1]-octane-2,4-dione (2.26 mmol) in methylene chloride (10 ml) containing triethylamine (0.47 ml, 3,39 mmol) is added dropwise at 0° a solution of acetyl chloride (0.27 g, 3.339 mmol) in 5 ml of methylene chloride. The resulting mixture is stirred for 30 min., and is then diluted with methylene chloride, washed, dried and evaporated to dryness to give 4-acetoxy-3-(4-chloro-2-nitrobenzoyl)-bicyclo[3,2,1]-diluted to give 4-acetoxy-3-(4-chloro-2-nitrobenzoyl)-bicyclo[3,2,1]-3-octen-2-one.

EXAMPLE 10

Following the procedure of Example 9, the final compounds under column I are prepared by the reaction of 3-(4-chloro-2-nitrobenzoyl)-bicyclo[3,2,1]octane-2,4-dione with the corresponding acyl chloride.

I

31. 4-propionyloxy-3-(4-chloro-2-nitrobenzyl)-bicyclo-[3,2,1]-3-octen-2-one
32. 2-isobutyryloxy-3-(4-chloro-2-nitrobenzoyl)-bicyclo-[3,2,1]-3-octen-2-one
33. 2-pivaloyloxy-3-(4-chloro-2-nitrobenzoyl)-bicyclo-[3,2,1]-3-octen-2-one
34. 2-benzoyloxy-3-(4-hloro-2-nitrobenzoyl)-bicyclo-[3,2,1]-3-octen-2-one
35. 4-pivaloyloxy-3-(4-chloro-2-nitrobenzoyl)-bicyclo[3,2,1]-3-octen-2-one (foam)

TABLE A

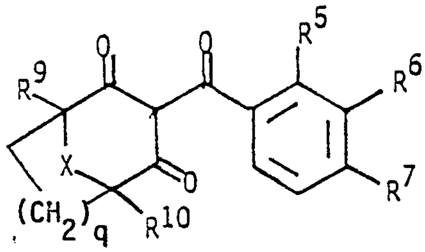

| Cpd | X | q | $R_9$ | $R_{10}$ | $R_5$ | $R_6$ | $R_7$ | m.p.(°C) |
|---|---|---|---|---|---|---|---|---|
| 1 | $CH_2$ | 1 | H | H | $NO_2$ | H | Cl | 134 |
| 2 | $CH_2$ | 1 | H | H | $NO_2$ | H | Br | 142 |
| 3 | $CH_2$ | 1 | H | H | Cl | H | $SO_2CH_3$ | foam |
| 4 | $CH_2$ | 1 | H | H | Cl | H | $OSO_2CH_3$ | |
| 5 | $CH_2$ | 1 | H | H | Cl | Cl | Cl | |
| 6 | $CH_2$ | 1 | H | H | Cl | Cl | $SO_2CH_3$ | |
| 7 | $CH_2$ | 1 | H | H | $CH_3$ | H | Br | 92-95 |
| 8 | $CH_2$ | 1 | H | H | $NO_2$ | H | $SO_2CH_3$ | 148 |
| 9 | $CH_2$ | 1 | H | H | $NO_2$ | $OCH_3$ | $OSO_2CH_3$ | |
| 10 | $CH_2$ | 1 | H | H | $NO_2$ | H | $OSO_2CH_3$ | 140 |
| 11 | $CH_2$ | 1 | H | H | $NO_2$ | $-O-CH_2-O-$ | | |
| 12 | $C(CH_3)_2$ | 1 | H | $CH_3$ | $NO_2$ | H | Cl | 101 |
| 13 | O | 1 | $CH_3$ | $CH_3$ | $NO_2$ | H | Cl | foam |
| 14 | O | 1 | $CH_3$ | $CH_3$ | $NO_2$ | H | Br | |
| 15 | O | 1 | $CH_3$ | $CH_3$ | $CH_3$ | H | Br | |
| 16 | O | 1 | $CH_3$ | $CH_3$ | Cl | Cl | Cl | |
| 17 | O | 1 | $CH_3$ | $CH_3$ | Cl | Cl | $SO_2CH_3$ | |
| 18 | O | 1 | $CH_3$ | $CH_3$ | Cl | H | $SO_2CH_3$ | |
| 19 | O | 1 | $CH_3$ | $CH_3$ | $NO_2$ | H | $SO_2CH_3$ | |
| 20 | O | 1 | $CH_3$ | $CH_3$ | $NO_2$ | $OCH_3$ | $OSO_2CH_3$ | |
| 21 | O | 1 | $CH_3$ | $CH_3$ | Cl | H | $OSO_2CH_3$ | |
| 22 | O | 1 | $CH_3$ | $CH_3$ | $NO_2$ | H | $OSO_2CH_3$ | |
| 23 | $CH_2$ | 1 | H | H | $NO_2$ | H | $SCH_2CH_3$ | 75 |
| 24 | $CH_2$ | 1 | H | H | $CH_3$ | H | Cl | 94 |
| 25 | $CH_2$ | 2 | H | H | $NO_2$ | H | Cl | 138 |
| 26 | $CH_2$ | 2 | H | H | $NO_2$ | H | F | 96 |
| 27 | $CH_2$ | 2 | H | H | $NO_2$ | H | $OSO_2CH_3$ | 167 |
| 28 | $CH_2$ | 2 | H | H | $NO_2$ | H | $SO_2CH_3$ | 160 |
| 29 | $CH_2$ | 2 | H | H | $NO_2$ | H | $SCH_3$ | 145-147 |
| 30 | $CH_2$ | 1 | H | H | $NO_2$ | H | $SCH_3$ | 126 |

Especially good activity has been demonstrated by Compound No. 30.

The starting compounds of formula II herein are known or, in cases where they are novel, can be produced by methods analogous to known methods or by methods described herein.

Thus, the enol esters of formula II can be prepared by the reaction of a 3,5-dione of formula III with a benzoyl halide of formula IV (wherein Q is a halogen atom) in the presence of a moderate base such as triethylamine.

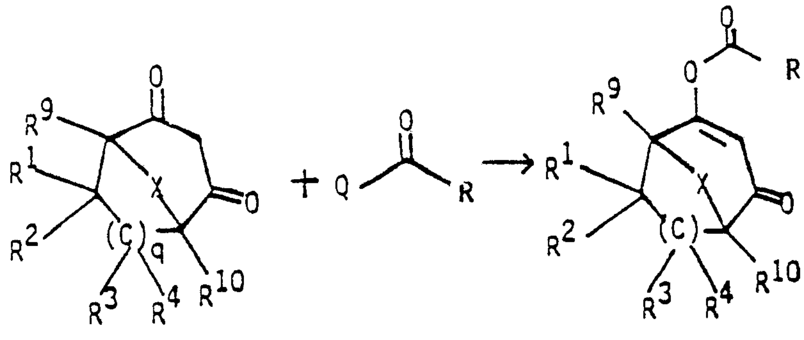

(III) (IV) (II)

The bicycloalkane diones of formula III can be synthesized by the methods such as a) described by Hiroshi Kakisawa et al., Bull. Chem. Soc. Jpn., 60:4369 (1987), or b) by oxidizing the known hydroxy-oxabicyclo[2,2,1]heptane (US 4,670,041) to oxabicyclo[2,2,1]heptane-2,3-diones. Ring expansion of oxabicyclo[2,2,1]heptane-2,3-dione with alkyl diazoacetate (described by Korobitsyna et al., J. Gen. Cheml USSR 27:1859 (1957)) followed by heating in the presence of water.

INTERMEDIATE COMPOUNDS

The following examples are presented to illustrate representative methods of preparing the intermediate compounds.

EXAMPLE 11

4-Chloro-2-nitrobenzoic acid (0.430 g, 2.13 mmol) is heated under reflux with thionyl chloride for 2 hr., after which excess thionyl chloride is removed under vacuum. The resulting acid chloride residue in 5 ml of methylene chloride is added to bicyclo[3,2,1]octane-2,4-dione (0.294 g, 2.13 mmol) dissolved in 10 ml of methylene chloride, with cooling to 0°, followed by dropwise addition of triethylamine (0.50 ml, 3.40 mmol). The mixture is stirred at RT for 30 min. and is then taken up in methylene chloride. The organic layer is washed with brine, dried and evaporated to give 4-(4-chloro-2-nitro-benzoyloxy)-bicyclo[3,2,1]-3-octen-2-one.

EXAMPLE 12

To a solution of (±) camphorquinone [(1,7,7)-trimethylbicyclo[3,2,1]heptane-2,3-dione] (15.0 g, 90.24 mmol) and ethyl diazoacetate (11.8 g, 103.7 mmol) in 120 ml of ether is added, at 0°, $BF_3$.ether (4 ml). The mixture is stirred at RT for 24 hr. The reaction mixture is extracted with 5% $K_2CO_3/H_2O$ until no polar ethyl carboxyl dione remains. The combined aqueous extracts are acidified with conc. HCl and extracted with ether. The combined organic extracts are dried and evaporated to dryness to give crystalline 3-carboethoxy-1,8,8-trimethylbicyclo[3,2,1]octane-2,4-dione.

The above octane-2,4-dione (5.0 g, 19.8 mmol) in 20 ml of dimethylsulfoxide (DMSO) and 0.8 ml of water is heated at 130-140° for 1 hr. The reaction mixture is diluted with ether, washed with water and with brine, dried and evaporated to give crystalline 1,8,8-tri-methylbicyclo[3,2,1]octane-2,4-dione, m.p. 220°.

EXAMPLE 13

To a solution of 1,8,8-trimethylbicyclo[3,2,1]octane-2,4-dione (2.0 g, 11.0 mmol) in 25 ml of methylene chloride containing triethylamine (2.30 ml, 16.0 mmol) is added, dropwise at 0°, a solution of 4-chloro-2-nitrobenzoyl chloride, prepared from the corresponding acid (2.23 g 11.0 mmol) and thionyl chloride, in methylene chloride (5 ml). After the addition is complete, the mixture is warmed to RT and stirred for 1 hr. at RT, after which it is diluted with methylene chloride, washed, dried and evaporated to dryness to give a mixture of 4-(4-chloro-2-nitrobenzoyloxy)-5,8,8-trimethyl-bicyclo[3,2,1]-3-octen-2-one and 4-(4-chloro-2-nitrobenzoyl-oxy)-1,8,8-trimethylbicyclo[3,2,1]-3-octen-2-one.

EXAMPLE 14

To a solution of 4-hydroxy-3-methoxy-2-nitrobenzaldehyde (10.0 g, 50.7 mmol) in 200 ml of methylene chloride and containing triethylamine (10.60 ml, 1.5 eq.) at 0° is added methanesulfonyl chloride (4.32 ml, 6.39 g, 1.1 eq.) over a period of 5-10 min. The mixture is stirred for an additional 10 min., and is then washed with ice water, with dilute HCl, with sat. $NaHCO_3$ and with brine. The layers are separated and the organic layer is dried and evaporated to give 3-methoxy-4-methylsulfonyloxy-4-nitrobenzaldehyde.

The above aldehyde (13.25 g) is suspended in acetone and cooled to 0°. Jones reagent is added dropwise over approx. 15 min. until the solution is slightly orange, indicating an excess of the reagent. The reaction is stirred for approx. 1 hr., after which it is diluted with water and extracted with ethylacetate to give 3-methoxy-4-methylsulfonyloxy-2-nitrobenzoic acid.

EXAMPLE 15

3-Methoxy-4-methylsulfonyloxy-2-nitrobenzoic acid (5.13 g, 17.6 mmol) is heated under reflux with thionyl chloride for 2 hr., after which excess thionyl chloride is removed under vacuum. The resulting acid chloride residue is added to bicyclo[3,2,1]octane-2,4-dione (17.6 mmol) dissolved in 50 ml of methylene chloride, with cooling to 5°, followed by dropwise addition of triethylamine (3.19 ml, 1.3 eq.). The mixture is stirred at RT for 2 hr. and then poured into water. The organic layer is washed with brine, dried and evaporated to give 4-(3-methoxy-4-methylsulfonyloxy-2-nitrobenzoyloxy)-bicyclo[3,2,1]-3-octen-2-one.

EXAMPLE 16

To a solution of 2-chloro-4-hydroxybenzoic acid (3.45 g, 20 mmol) and sodium hydroxide (2.40 g, 60.0 mmol) in 30 ml of water is added, dropwise at 0°, methanesulfonyl chloride (2.57 ml, 3.80 g, 33.0 mmol). After addition is complete, the reaction mixture is stirred at RT for 30 min., after which it is poured into water, acidified with dil. HCl and extracted with ether. The combined organic extracts are washed with brine, dried and evaporated to give 2-chloro-4-methylsulfonyl-oxybenzoic acid.

EXAMPLE 17

To a solution of bicyclo[3,2,1]octane-2,4-dione (14.0 mmol) in 20 ml of methylene chloride containing triethylamine (2.90 ml, 20.8 mmol) is added, dropwise at 0°, a solution of 2-chloro-4-methylsulfonyloxybenzoyl chloride, prepared from the corresponding acid (3.50 g, 14.0 mmol) and thionyl chloride, in methylene chloride (10 ml). After the addition is complete, the mixture is stirred for another 30 min. at 0°, after which it is diluted with methylene chloride, washed, dried and evaporated to dryness to give 4-(2-chloro-4-methylsulfonyloxy-benzoyloxy)-bicyclo[3,2,1]-3-octen-2-one.

EXAMPLE 18

Silver oxide is prepared by adding a solution of silver nitrate (43.53 g, 0.26 mol) in 100 ml of water to a solution of sodium hydroxide (20.48 g 0.51 mol) in 100 ml of water. To this brown semisolid mixture is added 6-nitropiperonal (25.0 g, 0.128 mol) in small portions with vigorous stirring. The reaction mixture is stirred for 2 hr. at 50°. The mixture is then filtered, and the filtrate is acidified, and extracted with ether. The ether extracts are dried and the ether evaporated off to give, after trituration with ether/hexane, 6-nitro-1,3-benzodioxole-5-carboxylic acid.

EXAMPLE 19

6-Nitro-1,3-benzodioxole-5-carboxylic acid (2.71 g) is heated under reflux with 25 ml of thionyl chloride for 2 hr., after which excess thionyl chloride is removed under vacuum. The resulting acid chloride residue is added to bicyclo[3,2,1]-octane-2,4-dione (11.8 mmol) dissolved in methylene chloride, with cooling to 5°, followed by dropwise addition of triethylamine (1.3 eq.). The mixture is stirred at RT for 2 hr. and then poured into water. The organic layer is washed with brine, dried and evaporated to give 4-(4,5-methylene-dioxy-2-nitrobenzoyloxy)-bicyclo[3,2,1]-3-octen-2-one.

**Claims**

1. A compound of the formula I

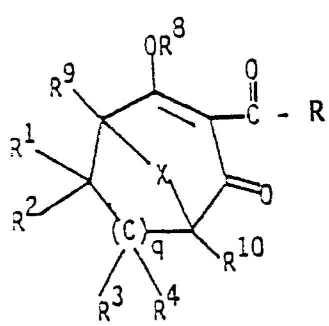

(I)

wherein R is selected from the groups

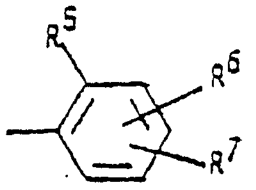

or

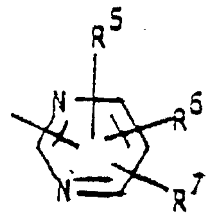

X is oxygen, sulfur or C1-4alkylene;
each of $R_1$, $R_2$, $R_3$, $R_4$, $R_9$ and $R_{10}$ is, independently, hydrogen, $C_{1-8}$alkyl or $COOR_{16}$;
$R_5$ is $C_{1-8}$alkyl, optionally substituted with one to six halogen atoms; $C_{1-8}$alkoxy, optionally substituted with one to six halogen atoms; $C_{1-8}$alkylcarbonyl; $C_{1-8}$alkoxycarbonyl; $NR_{13}R_{14}$; $O_nS(O)_{n'}R_{12}$; halogen; $NR_{15}SO_2R_{12}$; cyano; or nitro;
each of $R_6$ and $R_7$ is independently hydrogen or selected from the values of $R_5$; or $R_6$ and $R_7$ together from the group -Y-W-Z-;
$R_8$ is hydrogen, $C_{1-8}$alkyl, $C_{1-8}$alkylcarbonyl, $C_{1-8}$alkoxycarbonyl $C(O)NR_{13}R_{14}$, $C_{1-8}$alkylsulfonyl, $P(O)$-$(OR_{11})_2$, $R_{13}P(O)OR_{11}$, benzoyl or a salt forming moiety;
$R_{11}$ is $C_{1-8}$alkyl;
$R_{12}$ is $C_{1-8}$alkyl, optionally substituted with one to six halogen atoms;
each of $R_{13}$, $R_{14}$, $R_{15}$ and $R_{16}$ is, independently, hydrogen or $C_{1-8}$alkyl;
each of $R_{17}$ and $R_{18}$ is independently hydrogen, halogen or $C_{1-8}$alkyl optionally substituted with one to six halogen atoms; or $R_{17}$ and $R_{18}$ together form an oxo group;
each of $R_{19}$ and $R_{20}$ is, independently, hydrogen, halogen or C1-8alkyl optionally substituted with one to six halogen atoms;
W is $-(CR_{17}R_{18})_t-(CR_{19}R_{20})_{t'}-$ or $SO_2$;
each of Y and Z is independently oxygen, sulfur, $SO_2$, C=O or $CR_{15}R_{16}$;
with the proviso that Y and Z are attached to adjacent carbons;
n is zero or one;
n′ is zero, one or two;
q is zero, one or two;
t is one or two; and
t′ is zero or one.

2. A compound according to Claim 1 wherein R is the group

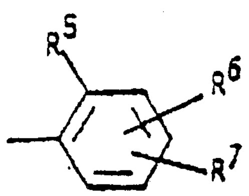

wherein $R_5$, $R_6$, and $R_7$ are as defined in claim 1.

3. A compound according to Claim 2 wherein $R_1$, $R_2$, $R_3$, $R_4$, $R_9$ and $R_{10}$ are independently hydrogen or $C_{1-4}$alkyl.

4. A compound according to Claim 3 wherein $R_5$ is selected from nitro, halogen, $C_{1-4}$alkyl, $R_6$ is selected from hydrogen, $C_{1-4}$alkoxy and halogen and $R_7$ is selected from halogen, $C_{1-3}$alkylsufonyloxy, $C_{1-3}$alkylsulfonyl and $C_{1-3}$alkylthio.

5. A compound according to Claim 4 wherein $R_8$ is hydrogen or a salt-forming moiety.

6. A compound of the formula

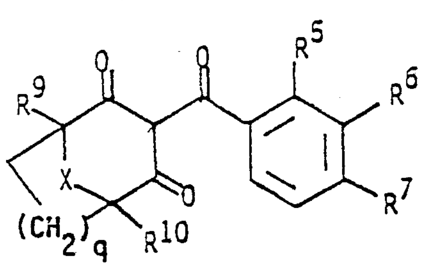

wherein q is 1, X is $CH_2$, $R_9$ is H, $R_{10}$ is H, $R_5$ is $NO_2$, $R_6$ is H and $R_7$ is $SCH_3$.

7. A herbicidal composition comprising the compound of formula I as defined in Claim 1, in association with an agriculturally acceptable carrier.

8. A method of controlling weeds comprising applying to the weeds or their locus an herbicidally

effective amount of a compound of formula I as defined in Claim 1.

9. A process of preparing a compound of formula I, stated in Claim 1, which comprises reacting an enol ester of formula II

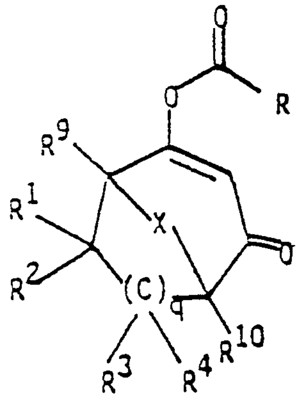

(II)

wherein R, x, q, $R_{1-4}$ and $R_{9-10}$ are as defined in claim 1, with a cyanide source and a moderate base to obtain the compound of formula I where $R_8$ is hydrogen, followed, where desired, by etherification or esterification to the corresponding enol ethers or enol esters.

10. A process according to Claim 9, substantially as described herein by way of Example.

11. A compound of formula I, whenever obtained by a process according to Claims 9 or 10.